# EUROPEAN PATENT SPECIFICATION

(11) **EP 1 784 276 B1**
(45) Date of publication and mention of the grant of the patent: **29.09.2010**
(21) Application number: 05806977.4
(22) Date of filing: 11.08.2005
(51) Int. Cl.: B23K 26/28, A61M 15/00, B05B 7/14, B65D 75/28, B65D 83/14

(54) **A METHOD OF FORMING A MEDICINAL AEROSOL FORMULATION RECEPTACLE CONTAINING A MEDICINAL AEROSOL FORMULATION THROUGH LASER WELDing A METAL FOIL**
VERFAHREN ZUR HERSTELLUNGS ÊINER AUFNAHME FÜR MEDIZINISCHE AEROSOLFORMULIERUNG MIT EINER MEDIZINISCHEN AEROSOLFORMULIERUNG MIT LASERGESCHWEISSUNG EINER METALLFOLIE
METHODE DE FABRICATION D'UNRECEPTACLE CONTENANT UNE FORMULATION MEDICINALE EN AEROSOL AVEC SOUDAGE LASER D'UNE FEUILLE METALLIQUE

(30) Priority: 23.08.2004 GB 0418738
(43) Date of publication of application: 16.05.2007
(73) Proprietor: 3M Innovative Properties Company, St. Paul, MN 55133-3427 (US)
(72) Inventor: WANG, Wei, Manchester Greater, Manchester M60 1QD (GB); LI, Lin, Manchester Greater, Manchester M60 1QD (GB); STEWART, Robert, Craigieburn, Victoria VIC 3064 (AU); HODSON, Peter, D., Loughborough Leicestershire 1EP (GB)
(74) Representative: Aleandri-Hachgenei, Lorraine E.
(86) International application number: PCT/US2005/028440
(87) International publication number: WO 2006/028639

(56) References cited:
- WO-A-02/092154
- WO-A-03/055547
- US-A- 4 137 914
- US-A- 4 493 574
- US-A- 5 096 518
- US-A1- 2001 029 948

## Description

This invention relates to receptacles for drug delivery and to their preparation. In particular the invention relates to Methods of forming hermetically sealed receptacles containing liquefied aerosol propellant medicinal aerosol formulation comprising liquefied aerosol propellant.

Pressurised metered dose inhalers have been used for over forty years for the treatment of asthma and other respiratory conditions. Pressurised metered dose inhalers comprise a container filled with many doses of propellant-based formulation, together with a metering valve for dispensing individual metered doses upon demand. One of the disadvantages of conventional metered dose inhalers is the difficulty in providing low numbers of doses e.g. less than thirty, in a cost effective manner with sufficient dose consistency.

Various other drug aerosolization devices are also known, such as dry powder inhalers, which sometimes use individual doses of formulation, and nebulizers.

US-A-4,137,914 discloses an inhaler for use with a capsule containing a single dose of drug and propellant. The capsule may be made of plastic or metal with the two parts either glued or fused together.

US 5,096,518 describes a method of encapsulating a material to be processed by warm or hot isostatic pressing which basically includes the steps of (a) enveloping a processing material in the form of loose or compacted powder with a metal foil of 30 microns thickness, and (b) welding overlying and underlying metal foil potions to encapsulate said material in said metal foil in sealed state,

Despite some advances in the field, there remains a constant challenge to provide hermetically sealed aerosol drug formulation container systems that are manufacturable at reasonable cost and meet stringent regulatory standards in terms of physical and chemical stability of the formulation, dose accuracy and consistency, as well as limiting contaminant and impurity levels. This can be particularly difficult for pressurized formulations such as those containing liquefied propellants.

It has now been found that medicinal aerosol formulations can be delivered from hermetically sealed receptacles formed using laser welding of metal foils. Particularly surprising is that propellant based medicinal aerosol formulations can be effectively contained in and delivered from such receptacles. According to the present invention there is provided a method of forming an hermetically sealed metal receptacle containing a liquefied aerosol propellant or a medicinal aerosol formulation comprising liquefied aerosol propellant wherein at least a portion of the receptacle comprises metal foil having a thickness of from 25µm to 250µm, wherein said method comprises chilling the receptacle to be sealed to maintain liquefied propellant or medicinal aerosol formulation comprising liquefied aerosol propellant therein while laser welding the metal foil of the receptacle to provide an hermetic seal.

Use of methods described herein advantageously allows the provision of hermetically sealed metal receptacles containing either liquefied aerosol propellant or pressurized medicinal aerosol formulation comprising liquefied aerosol propellant.

The use of the metal foil has the advantage that it provides a convenient means of access to the receptacle as it may be pierced, thereby obviating the need of providing the receptacle with a valve, e.g. as an access and/or closure means for filling and/or dispensing material into or from the inferior of the receptacle, and thus avoiding a number of issues associated with the use of such dispensing valves, such as moisture and/or air ingress over long storage periods, and unwanted interaction of components with elastomeric seals used in such dispensing valves. Thus, the receptacles are advantageously free of elastomeric seals and/or dispensing valves.

Additionally the metal-to-metal laser welded sealing of the receptacle advantageously avoids potentially undesirable interaction of the contained medicinal aerosol formulation or components thereof with adhesive compositions or components thereof. Thus, the receptacles are advantageously are free of adhesives at the seal, more particularly the receptacles are essentially free or free of non-metallic components at the seal. Furthermore, the formation of the hermetic seal through laser welding, more particularly pulsed laser welding, advantageously provides a robust receptacle for handling, storage, and especially, for accessing the material in the receptacle through piercing, e.g. such that the seal does not break under the mechanical stress of such piercing into the metal foil of the receptacle.

For enhanced robustness of the receptacle the metal foil desirably has a thickness of at least 38µm, more desirably of at least 50µm. To further facilitate access to the receptacle e.g. through piercing, the metal foil desirably has a thickness of at most 150µm, more desirably of at most 100µm, most desirably of at most 75µm.

The dimensions of the receptacle may be varied depending upon the intended use of the contents. For example, the receptacle may have a large volume to act as a reservoir of propellant or medicinal aerosol formulation. However, such receptacles provide particularly advantageous, convenient means by which a limited number of doses (thirty or less) and more particularly individual doses may be separately hermetically sealed and stored and from which they may be subsequently dispensed. The provision of a limited number of doses and more particularly the provision of individually contained pre-metered doses in this way can provide benefits with drugs that are highly moisture sensitive, or expensive, etc. The receptacle may be in the form of a pouch, vial or the like. Thus the receptacle may have an internal volume of 2 ml or less, more desirably 1 ml or less, even more desirably 0.5 ml or less. To accommodate a single dose of a medicinal aerosol formulation or a single charge of aerosol propellant for firing an aerosol device, such as an inhaler, the receptacle desirably has an internal volume of 0.2 ml or less, more desirably 0.15 ml or less and most desirably about 0.15 ml.

As detailed in the following, it has been found that the provision of a laser welded seal, more particular a pulsed laser welded seal, especially in the form of a weld comprising a plurality of connected weld beads formed by pulsed laser welding is particularly advantageous in terms of robustness and in terms of allowing the sealing of receptacles, in particular low volume receptacles (from 2 ml down to 0.10 ml) containing liquefied aerosol propellant and/or medicinal aerosol formulations, especially pressurized medicinal aerosol formulations, via laser welding thin metal foils. Moreover, it has surprisingly been found that it is possible to chill the receptacle to be sealed sufficiently to maintain liquefied propellant and/or a medicinal aerosol formulation comprising a liquefied propellant therein, and to accomplish laser welding, in particular pulsed laser welding, of the metal foil of the receptacle to provide an hermetic seal even when such receptacle has an internal volume of 2 ml down to 0.10 ml. Remarkably the laser welding is unaffected by the close proximity of the chilling medium, cold liquefied propellant and/or the chilling of the respective parts being welded. This is particularly surprising since simply the welding of thin metal foils in itself - let alone for hermetically sealing liquefied aerosol propellant or a pressurized medicinal aerosol formulation within a receptacle - by laser welding techniques is generally recognized as extremely difficult, as discussed in e.g. US-5,502,292 and US-A-4,798,931.

In preferred embodiments, the receptacle comprises a metal body defining an aperture and having a planar flange, and the metal foil extends over the aperture and is hermetically sealed to the flange.

Accordingly in a preferred embodiment of the present invention the method of forming a hermetically sealed receptacle containing liquefied aerosol propellant or medicinal aerosol formulation comprising liquefied aerosol propellant further comprises the steps:
providing a metal body defining an aperture and having a planar flange;
cooling and maintaining the metal body at a temperature below the boiling point of the aerosol propellant or the medicinal aerosol formulation comprising aerosol propellant to be sealed into the receptacle;
introducing the liquefied aerosol propellant or the medicinal aerosol formulation comprising liquefied aerosol propellant into the metal body;
positioning a metal foil having a thickness of from 25µm to 250µm over the aperture and the flange;
holding the metal foil against the flange; and
welding the metal foil to the flange using a pulsed laser to form a plurality of connected weld beads around the flange thereby forming the hermetic seal; and wherein the step of introducing is performed at least subsequent to the providing metal body step and at least prior to the holding step. In other words, the step of introducing may be performed before or after the step of positioning.

The step of cooling is performed at least subsequent to the providing the metal body step, and the introducing step is performed at least subsequent to the cooling step and at least prior to the holding the metal foil step. In other words the cooling step may be performed before or after the positioning of metal foil step. Also the introducing step, which in every case occurs at some time subsequent to the cooling step, may also be performed before or after the positioning of metal foil step, as the case may be, but at least prior to the holding of metal foil step.

The metal body generally has a thickness of at least 25 µm. Generally the metal body has a thickness of at most 300µm. The metal body and the metal foil may be two separate components or they may be integrally formed. For pouch or pillow-like receptacles, the metal body may have a thickness similar to that for the metal foil. The metal body desirably has a thickness of at least 38µm, more desirably of at least 50µm. The metal body desirably has a thickness of at most 250µm, more desirably at most 1,50µm, even more desirably of at most 100µm, most desirably of at most 75µm. For vial-like receptacles, the metal body may have a thickness greater than that of the metal foil, desirably at least 200µm. In general, to further facilitate the formation of a hermetically sealed receptacle and robustness of the sealed receptacle, the metal body desirably has a thickness of at least 200µm. The planar flange provides a surfaces for placement and welding of a metal foil extending across the aperture. The flange also provides a surface against which the foil is held, typically in intimate contact, during the welding process.

The mental body and foil may be made of any convenient metal e.g. aluminium, stainless steel etc. Stainless steel is preferred.

Other embodiments in accordance to the present invention are defined in the dependent claims.

In the subsequent discussion reference will be made to the accompanying drawings in which:
Figure 1 provides a schematic, cross-sectional illustration of an exemplary, reference assembly for use in laser welding a metal foil on a metals body;
Figure 2 represents an image of a photograph taken through an optical microscope of the external view of a portion of an exemplary pulsed-laser welded seal in accordance to the present invention; and
Figure 3 provides a schematic, cross-sectional illustration of an exemplary assembly for use in laser welding a metal foil on a metal body in accordance to the present invention.

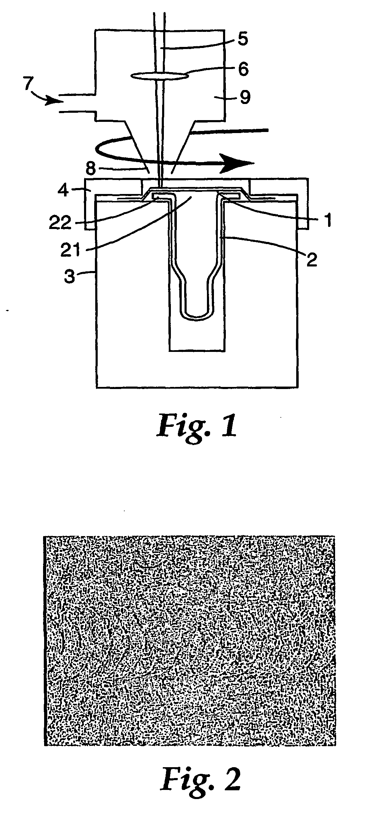

Referring to Figure 1 showing a schematic, cross-sectional illustration of an exemplary assembly for use in an exemplary method of laser welding a metal foil (1) on a metal body (2), the metal body may be positioned on a holder (3), e.g. by insertion into a channel of a holding block.

As can be see from Figure 1, the metal body (2) typically defines an aperture (21) and has a planar flange (22).

Generally the planar flange is near or adjacent to the aperture or surrounding the aperture. The metal body and the metal foil may be two separate components (as illustrated e.g. in Figure 1) or they may be integrally formed. In the latter case, the metal foil may for example be formed as an extension of the metal body, e.g. from a portion of the aperture rim in the form of a lid positioned over the aperture and the flange or capable of being positioned (e.g. by folding) over the aperture and the flange of the metal body. The metal body may be conveniently formed by any suitable technique, including casting, moulding, or deep drawing. The flange may extend radially inwardly or radially outwardly of the aperture and preferably has a radial width of at least 1 mm, more preferably at least 2mm, most preferably at least 3mm. Where the flange extends radially inwardly, the metal body may preferably be formed from two pre-welded parts.

In order to facilitate the successful formation of a hermetic weld-seal, it is desirable to ensure that the surfaces to be welded are smooth, flat and held together in such a way as not to buckle under the influence of heat. Preferably, the surface of the flange of the metal body to which the metal foil is to be welded is ground and polished to a surface roughness of about 1 µm. Also it is desirable to minimize or eliminate any gap between surfaces of the foil and flange to be welded, to facilitate and enhance the conduction of heat during welding in order to provide an adequate molten region on the foil and flange and at the same time to help avoid rupture of the molten foil.

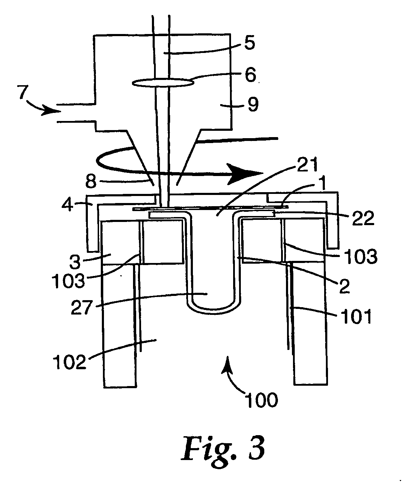

Clamps may be used to hold the metal foil and the flange in intimate contact as close as possible to the weld line. Preferably the edges of the clamps come to within 0.5mm of the centre of the weld line. The clamps (4) may be mechanical, for example as shown in Figures 1 and 3, however the use of magnetic clamps is preferred, in order to avoid the need for time-consuming clamp bolting procedures. For example, the metal body holder and the clamps could be made of ferromagnetic ferritic stainless steel. The metal foil may be held against the flange by other means, for example by fixing, at least temporarily, the metal foil to the flange through e.g. cold-welding.

The metal foil is laser welded, more desirably pulsed laser welded, to form the hermetic seal. In providing the seal, typically a continuous welded seal, desirably the laser is pulsed while moving it relative to the parts to be sealed, to provide a weld comprising a plurality of connected weld beads. Again referring to Figure 1 as an example, the laser beam (5), typically arriving via an optical cable, may be focussed by a lens (6) or plurality of lenses provided for example in a housing (9) with an outlet (8) positioned above the upper surface of the metal foil (1). As discussed in more detail below, a shrouding gas may be used, and thus the housing (9) may also be provided with an inlet (7) for the supply of such a shrouding gas, which will also exit the housing at the outlet (8). As shown in Figure 1, the laser beam together with its housing can be rotated in a manner generally shown by the hollow arrow, so that the centre of the projected laser beam (5) substantially describes a circle on the upper surface of the metal foil (1). As will be appreciated by those skilled in the art, the laser beam may be moved in any manner, in a circle, ellipse, arc, straight-line, rectangle etc. necessary to provide the appropriate welded seal. Alternatively but less preferably, the laser beam with its housing may be held fixed while rotating or moving the holder (3) together with the metal body (2) and the metal foil (1) clamped thereto.

The use of pulsed laser welding to provide a hermetic seal in the form of a weld comprising a plurality of connected weld beads has been found to be preferable to the use of a continuous, non-pulsed laser welding, because resolidification of material after a pulse or several pulses of laser leads to a weld bead that holds together in intimate contact the metal body and foil regions next to be welded. Whereas when a non-pulsed laser beam is used, thermal distortion of the foil caused by the high thermal gradients typically leads to "bum through" of the foil, rather than welding.

A preferred source of pulsed laser is a Nd: YAG (neodymium yttrium aluminium garnet) laser.

To advantageously avoid rupture or cutting through the metal foil during pulsed laser welding, it is desirable to keep the area of molten film small relative to its thickness and/or to avoid excessive vaporisation of metal. It has been found advantageous to use pulse energies less than 0.5 J, more desirably from 0.1 to 0.4 J, most desirably from 0.2 to 0.3 J. Furthermore, it has been found advantageous to use a relatively short pulse width (duration), desirably less than 0.5 msec, more desirably from 0.1 to 0.45 msec, even more desirably 0.25 to 0.35 msec, most desirably about 0.3 msec. Preferably the peak power is at most 1000 W, more desirably from 200 to 1000 W, even more desirably 425 to 1000W, most desirably from 650 to 1000W.

Surprisingly it has been found that it is possible by employing high laser pulse frequencies (e.g. about 200 pulses/sec or more) to weld hermetic seals at high welding speeds (e.g. about 24 mm/sec or faster), which is particularly advantageous for e.g. on-line manufacturing as well as in sealing low volume receptacles containing liquefied propellant. The particular weld speed applied depends in part to the laser beam diameter used (which in terms of non-cutting or rupturing the metal foil is preferably 0.6 mm or lower either direct or after focussing through a lens) as well as the applied frequency of laser pulses. It has been found desirable to use at least about 4.25 pulses or more (more desirably about 6 pulses or more, e.g. from 6 to 25 pulses) per selected beam diameter. For example with a selected beam diameter of 0.2 mm, a weld speed of 24 mm/sec together with a pulse frequency of 510 pulses/sec provides 4.25 pulses per 0.2 mm diameter, while for a selected beam diameter of 0.5 mm, a weld speed of 60 mm/sec together with a pulse frequency of 1000 pulses/sec provides 8.3 pulses per 0.5 mm diameter. Weld speeds of 30 mm/sec or more, 60 mm/sec or more, and even 80 mm/sec or more have been achieved. Pulse frequencies of 200 or more, 500 or more, or 800 or more may be used. The upper limit of suitable pulse frequency may be in part dictated by the particular laser being used. However pulse frequencies up to 1000 pulses/sec have been here successfully applied, and it is expected that higher pulse frequencies would be suitable.

Figure 2 shows an image of an exemplary continuous weld seal of a 38 µm thick, annealed AISI 316L foil onto a 200 µm thick annealed AISI 316L flange using a 400 W Nd:YAG laser (0.6 mm beam diameter and 1.064 µm wavelength) with a pulse energy of 0.27 J (using a peak power of 900 W and a pulse width of 0.3msec) and a welding speed of 36 mm/sec in conjunction with a pulse frequency of 1000 pulses/sec. As can be see in Figure 2, the welded seal or the weld comprises a plurality of connected weld beads, in particular a series of overlapping weld beads with a stitched appearance along the path of the weld.

As mentioned above, for forming a hermetically sealed receptacle containing liquefied aerosol propellant or a medicinal aerosol formulation comprising liquefied aerosol propellant, the method desirably further includes the steps: cooling and maintaining the metal body at a temperature below the boiling point of the aforesaid aerosol propellant; and introducing liquefied aerosol propellant and/or a medicinal aerosol formulation comprising liquefied aerosol propellant into the metal body.

Referring to Figure 3 showing a schematic, cross-sectional illustration of an exemplary assembly for use in an exemplary method of forming such a receptacle, a chilling unit (100) is provided, for example a bath (101) containing a refrigerated liquid (102) which is maintained below the boiling point of the aerosol propellant or the aerosol propellant of a medicinal aerosol formulation to be sealed. In the exemplary arrangement illustrated in Figure 3, the bath (101) can be appropriately mounted in the holder (3) equipped with vents (103). Similar to the exemplary arrangement illustrated in Figure 1, the metal body (2) may then be positioned with the holder (3) and now at the same time into refrigerated liquid (102) so that the metal body is cooled and maintained at a temperature below the boiling point of the aerosol propellant, e.g. HFA 134a (boiling point -26.1 °C) or HFA 227 (boiling point - 16.5°C). Liquefied aerosol propellant or a medicinal aerosol formulation comprising liquefied aerosol propellant (10) then is introduced into the metal body (2). The metal foil (1) may be then positioned across the aperture (21) and the flange (22), and held against the flange through clamps (4). Alternatively the metal foil may be positioned over the aperture and the flange of the metal body prior to introducing contents into the metal body or even prior to cooling the metal body, for example where the contents are introduced after cooling of the metal body through an inlet tube located between the foil and metal body, the tube being removed prior to holding the foil against the flange. Similar to the exemplary arrangement illustrated in Figure 1, the metal foil (1) is then welded to the flange (22) by rotating the laser beam as indicated by the hollow arrow. Here it is desirable to rotate or move the components associated with the laser beam during welding to prevent spilling of liquid out of the metal body.

As indicated above, it is surprising especially for sealing low volume receptacles that the laser welding is unaffected by the close proximity of chilling medium, cold liquefied propellant and/or the chilling of the respective parts being welded, even at the temperatures needed to fill and seal liquefied HFA 134a and/or HFA 227 based medicinal formulations. Furthermore, it is remarkable, again especially for sealing low volume receptacles, that the heat produced from such laser welding as described herein is so localised that, when a receptacle of cold liquid is so sealed, the liquid hardly experiences any change in temperature.

For such welding in the vicinity of low temperature, it has been found advantageous to use a dry shrouding/shielding gas, such as dry nitrogen, argon or helium, in particular dry nitrogen or dry argon, during laser welding in order to prevent moisture condensation and frosting. The use of a dry shrouding gas also facilitates intimate contact between the metal parts to be welded and the avoidance of the generation of unwanted steam at the melt pool, as well as presenting moisture ingress into medicinal aerosol formulations that may be sensitive to moisture. In additional the use of oxygen-free dry shrouding/shielding gas, such as oxygen-free dry nitrogen, during laser welding also helps to ensure that the medicinal aerosol formulations to be sealed are maintained in an environment free of oxygen as well as moisture, and thus again protecting the stability of the pharmaceutical product. The flow rate of shrouding gas for such purposes is typically 5 liters/minute or more, e.g. 5 to 15 liters/minute.

It has also been found advantageous to apply higher flow rates (e.g. 60 liters/minutes or more) of shrouding gas in order to facilitate holding of the metal foil in certain situations. Moreover, to seal a receptacle, it is may be necessary to make the continuous weld describing a closed path e.g. a circle, ellipse, rectangle etc. While it may be desirable to clamp the parts to be welded at both sides of the weld line, when a closed path is to be welded any fixed clamp at the inner side of the weld line would obstruct the laser beam at some point unless the beam is re-angled during its transit and/or the clamp is transparent to the laser beam. Also mechanical clamping on both sides of the weld line may not be at all possible in certain situations, e.g. when the weld line is positioned close to the edge of the aperture, as is desirable to prevent or minimize the formation of internal crevices in which formulation constituents could subsequently become trapped. Also another difficulty is that often the unsupported part of the foil above the aperture will not withstand mechanical clamping pressure. Here in such situations it has been found that restraining the central part of the foil by providing sufficient flow of shrouding gas to apply adequate and uniform pressure against the foil towards the rim of the aperture greatly enhances the reliability of the seal. For such holding or restraining, the flow rate of shrouding gas is desirably at least 60 liters/minute, more desirably from 60 to 90 liters/minutes. A coaxial nozzle diameter of 3mm around the laser beam was found to be adequate to spread out this gas flow evenly. The gas flow exerts a downward pressure on the top foil layer, pressing it into intimate contact with the flange and thereby enhancing good contact and consistent welding as well as minimizing undesirably stress or distortion of the metal foil. Surprisingly, such a high gas flow does not cause troublesome vibrations in the thin top foil, as might be expected, nor does it cause melt ejection and consequent risk of cutting the foil.

While closed welding paths having corners can be achieved, generally circular-like closed welding paths are preferred in order to avoid the need to address and control heating and cooling rates at weeding path corners.

As stated above, stainless steel is the preferred material for the metal bodies and/or the mental foils. Its advantages (e.g. compared with aluminium) include high strength, low reflectivity to Nd:YAG laser light, low thermal conductivity, inertness to typical medicinal aerosol formulations, and ease of welding. More preferred are grades of stainless steel having a low carbon content of at most on 12%, even more preferred at most 0.08 %; most preferred are grades having at most 0.03% carbon, such as AISI316L, AISI304L and AISI317L. Such grades of stainless steel show good corrosion resistance after wending, because their low carbon content prevents or reduces chromium carbide precipitation at grain boundaries during resolidification, and thus prevents or reduces consequent chromium depletion elsewhere and consequent loss of the passivating chromium oxide surface layer.

The invention will be illustrated by the following Examples.

### Reference Example 1

The arrangement used for laser welding metal foils onto metal bodies in this example is schematically illustrated in Figure 1.

The metal body (2) was formed out of AISI 305 S19 stainless steel by deep drawing to a thickness of around 250 µm into the shape of a test-tube having a more slender cross section at its lower end and an annular flange oriented radially outwardly about the upper open end. The internal volume was 200 microlitres and the flange width 1.14 mm. Each flange was ground and polished to a surface roughness of about 1 µm, the thickness of the flange being at least 200 µm after the grinding. The metal body was inserted into a channel of a holder (3) in the form of a solid steel block. A 50 µm AISI 316L annealed stainless steel foil, supplied by Goodfellow Cambridge Ltd, part no. FF210250, was placed over the open end of the metal body, and held down by means of a clamping device (4) that was screwed down to the solid steel block (3). A 400W Scorpion Nd:YAG laser was used having a 0.6 mm beam core diameter, and set to emit square-wave 0.3 ms pulses of laser light of wavelength 1.064 µm. The laser beam (5) arriving via an optical cable with a 0.6 mm core, was focussed to around 0.5 mm diameter by a lens arrangement (6) and surrounded by a housing with an inlet (7), for supply of shrouding gas of dry oxygen-free nitrogen at between 60 and 90 litres per minute, and a converging outlet (8). The exit of the convergent outlet was 3 mm in diameter and positioned 4 mm from the upper surface of the foil. The laser housing was mounted on a 3-axis CNC table so that it could be rotated in a manner generally shown by the hollow arrow, so that the centre of the projected laser beam substantially described a circle having a diameter of 6.8 mm on the upper surface of the foil. The welding parameters used for a set of 7 experiments are detailed in Table 1 (Examples 1(a) to (g); 4 to 6 individual samples welded per each condition). Out of 39 samples prepared by this process, all were successful.

**Table 1. Welding parameters**

| **Conditions** | **Average power (W)** | **Peak power (kW)** | **Pulse energy (J)** | **Pulse frequency (kHz)** | **Welding speed (mm/s)** |
|---|---|---|---|---|---|
| 1 (a) | 216 | 0.9 | 0.27 | 0.8 | 60 |
| 1 (b) | 243 | 0.9 | 0.27 | 0.9 | 60 |
| 1 (c) | 270 | 0.9 | 0.27 | 1 | 60 |
| 1 (d) | 270 | 0.9 | 0.27 | 1 | 48 |
| 1 (e) | 270 | 0.9 | 0.27 | 1 | 36 |
| 1 (f) | 270 | 0.9 | 0.27 | 1 | 42 |
| 1 (g) | 210 | 0.7 | 0.21 | 1 | 36 |

### Example 2

Metal bodies were formed out of AISI 305 S19 stainless steel by deep drawing to a thickness of 250 µm into the shape of a test-tube having an internal cross section of 5 mm diameter, an internal length of 7mm and a flange of width 2.5 mm at its upper open end. The metal body had an internal volume of 135 microlitres. The flange top surface was ground and polished to a surface roughness of about 1 µm and a thickness no less than 200 µm. Metal bodies were placed in a holder and filled to about half full with propellant from an inverted "Air Duster" 400 ml Ultrajet Airduster, part number ES1520E, ITW- Chemtronics, Rocol House, Swillington, Leeds, LS26 8BS, West Riding of Yorkshire, UK. Supplied via RS Components Ltd, P.O.Box 99, Corby, Northamptonshire, NN17 9RS, UK; order code 388-8718. The propellant in the 'air duster' comprises 90-100% 1,1,1,2-tetrafluoroethane plus 2-6% dimethyl ether. This was achieved by slowly dispensing liquid from the inverted 'air duster' to both cool the metal body and dispense propellant into it. The contents of the metal bodies were consequently at a temperature of about -27°C. In this example liquefied propellant was sealed within receptacles using parameters as in Example 1 and summarized in Table 2.

**Table 2. Welding process operating parameters**

| | |
|---|---|
| Laser type | 400W Scorpion Nd:YAG laser |
| Laser pulse shape | Square |
| Wavelength | 1.064 µm (near-infrared) |
| Peak power of laser pulse, (W) | 700 - 900 |
| Frequency of laser pulse, (Hz) | 800 - 1000 |
| Laser pulse width, (ms) | 0.3 |
| Welding speed, (mm/s) | 36 - 60 |
| Shrouding gas | Nitrogen |
| Shrouding gas flow rate, (Umin) | 60 - 90 |

The arrangement used for laser welding in this example was similar to the arrangement used in Example 1, except that the flange was wide enough to be enveloped by the clamp (as shown in Figure 3) that was arranged to be held in place magnetically for speed of operation.

Surprisingly, it was found that the laser processing conditions did not need to be changed, although the metal bodies and foils had been chilled to a temperature below the boiling point of HFA 134a.

## Claims

1. A method of forming a hermetically sealed receptacle containing a liquefied aerosol propellant or a medicinal aerosol formulation comprising liquefied aerosol propellant (10) **characterized in that** at least a portion of the receptacle comprises metal foil (1) having a thickness of from 25µm to 250µm, wherein said method comprises chilling the receptacle to be sealed to maintain liquefied aerosol propellant or medicinal aerosol formulation comprising liquefied aerosol propellant therein while laser welding the metal foil of the receptacle to provide an hermetic seal.

2. A method of forming a hermetically sealed receptacle as claimed in claim 1, in which the welding of the metal foil (1) is performed using a pulsed laser (5) to form a plurality of connected weld beads thereby forming the hermetic seal.

3. A method of forming a hermetically sealed receptacle as claimed in claim 1 or claim 2, the method comprising the steps:
providing a metal body (2) defining an aperture (21) and having a planar flange (22);
cooling and maintaining the metal body (2) at a temperature below the boiling point of the aerosol propellant or the medicinal aerosol formulation comprising aerosol propellant to be sealed into the receptacle
introducing the liquefied aerosol propellant or the medicinal aerosol formulation comprising liquefied aerosol propellant (10) into the metal body (2);
positioning the metal foil (1) over the aperture (21) and the flange (22);
holding the metal foil (1) against the flange (22); and
welding the metal foil (1) to the flange (22) using a pulsed laser (5) to form a plurality of connected weld beads around the flange (22) thereby forming the hermetic seal, wherein the step of introducing is performed at least subsequent to the providing metal body (2) step and at least prior to the holding step, and wherein the cooling step is performed at least subsequent to the providing the metal body (2) step, and the introducing step is performed at least subsequent to the cooling step and at least prior to holding the metal foil (1) step.

4. A method of forming a hermetically sealed receptacle as claimed in any preceding claim in which the aerosol propellant is selected from HFA 134a, HFA 227 and mixtures thereof.

5. A method of forming a hermetically sealed receptacle as claimed in any preceding claim in which a shrouding gas is provided around the weld during welding.

6. A method of forming a hermetically sealed receptacle as claimed in claim 5 as dependent directly or indirectly on claim 3 in which the shrouding gas is impinged onto the foil (1) and directed to hold the foil in intimate contact with the flange (22).

7. A method of forming a hermetically sealed receptacle as claimed in any preceding claim, wherein the internal volume of the sealed receptacle is 2 ml or less.

8. A method of forming a hermetically sealed receptacle as claimed in any preceding claim, wherein the metal foil (1) is made of metals selected from aluminium and stainless steel.

9. A method of forming a hermetically sealed receptacle as claimed in one claims 3 to 8, wherein the metal body (2) is made of metals selected from aluminium and stainless steel.

10. A method of forming a hermetically sealed receptacle as claimed in claim 9, wherein the metal body (2) and foil (1) are made of stainless steel.

## Patentansprüche

1. Verfahren zur Ausbildung eines hermetisch versiegelten Behälters, der ein verflüssigtes Aerosoltreibmittel oder eine medizinische Aerosolformulierung, die verflüssigtes Aerosoltreibmittel (10) umfasst, enthält, **dadurch gekennzeichnet, dass** mindestens ein Teil des Behälters Metallfolie (1) mit einer Dicke von 25 µm bis 250 µm umfasst, wobei das Verfahren das Kühlen des zu versiegelnden Behälters zum Erhalten des verflüssigten Aerosoltreibmittels oder der medizinischen Aerosolformulierung, die verflüssigtes Aerosoltreibmittel umfasst, darin, während die Metallfolie des Behälters unter Bereitstellung einer hermetischen Versiegelung lasergeschweißt wird, umfasst.

2. Verfahren zur Ausbildung eines hermetisch versiegelten Behälters nach Anspruch 1, bei dem die Metallfolie (1) unter Verwendung eines gepulsten Lasers (5) zur Ausbildung mehrerer verbundener Schweißnähte geschweißt wird, wodurch sich die hermetische Versiegelung bildet.

3. Verfahren zur Ausbildung eines hermetisch versiegelten Behälters nach Anspruch 1 oder 2, wobei das Verfahren die folgenden Schritte umfasst:
Bereitstellen eines Metallkörpers (2), der eine Öffnung (21) definiert und einen planaren Flansch (22) aufweist;
Kühlen und Halten des Metallkörpers (2) bei einer Temperatur, die unter dem Siedepunkt des in dem Behälter zu versiegelnden Aerosoltreibmittels oder der in dem Behälter zu versiegelnden medizinischen Aerosolformulierung, die Aerosoltreibmittel umfasst, liegt;
Einbringen des verflüssigten Aerosoltreibmittels oder der medizinischen Aerosolformulierung, die verflüssigtes Aerosoltreibmittel (10) umfasst, in den Metallkörper (2);
Positionieren der Metallfolie (1) über der Öffnung (21) und dem Flansch (22);
Halten der Metallfolie (1) gegen den Flansch (22); und
Schweißen der Metallfolie (1) an den Flansch (22) unter Verwendung eines gepulsten Lasers (5) unter Ausbildung mehrerer verbundener Schweißnähte um den Flansch (22) herum, wodurch sich die hermetische Versiegelung bildet, wobei der Schritt des Einbringens mindestens auf den Schritt des Bereitstellens des Metallkörpers (2) folgend und mindestens vor dem Schritt des Haltens stattfindet, und wobei der Schritt des Kühlens mindestens auf den Schritt des Bereitstellens des Metallkörpers (2) folgend stattfindet und der Schritt des Einbringens mindestens auf den Schritt des Kühlens folgend und mindestens vor dem Schritt des Haltens der Metallfolie (1) stattfindet.

4. Verfahren zur Ausbildung eines hermetisch versiegelten Behälters nach einem vorhergehenden Anspruch, bei dem das Aerosoltreibmittel ausgewählt ist unter HFA 134a, HFA 227 und deren Mischungen.

5. Verfahren zur Ausbildung eines hermetisch versiegelten Behälters nach einem vorhergehenden Anspruch, bei dem ein Mantelgas während des Schweißens um die Schweißung bereitgestellt wird.

6. Verfahren zur Ausbildung eines hermetisch versiegelten Behälters nach Anspruch 5, wie er direkt oder indirekt von Anspruch 3 abhängig ist, bei dem das Mantelgas auf die Folie (1) auftrifft und so geführt wird, dass es die Folie in innigem Kontakt mit dem Flansch (22) hält.

7. Verfahren zur Ausbildung eines hermetisch versiegelten Behälters nach einem vorhergehenden Anspruch, wobei das Innenvolumen des versiegelten Behälters 2 ml oder weniger beträgt.

8. Verfahren zur Ausbildung eines hermetisch versiegelten Behälters nach einem vorhergehenden Anspruch, wobei die Metallfolie (1) aus Metallen ausgewählt unter Aluminium und rostfreiem Stahl besteht.

9. Verfahren zur Ausbildung eines hermetisch versiegelten Behälters nach einem der Ansprüche 3 bis 8, wobei der Metallkörper (2) aus Metallen ausgewählt unter Aluminium und rostfreiem Stahl besteht.

10. Verfahren zur Ausbildung eines hermetisch versiegelten Behälters nach Anspruch 9, wobei der Metallkörper (2) und die Folie (1) aus rostfreiem Stahl bestehen.

## Revendications

1. Procédé de fabrication d'un réceptacle hermétiquement étanche contenant un gaz propulseur en aérosol liquéfié ou une formulation médicinale en aérosol contenant le gaz propulseur en aérosol liquéfié (10), **caractérisé en ce qu'**au moins une partie du réceptacle comprend une feuille métallique (1) qui présente une épaisseur comprise entre 25 µm et 250 µm, dans lequel ledit procédé comprend la réfrigération du réceptacle à sceller afin de maintenir le gaz propulseur en aérosol liquéfié ou la formulation médicinale en aérosol contenant le gaz propulseur en aérosol liquéfié dans celui-ci pendant le soudage au laser de la feuille métallique du réceptacle dans le but de former un joint hermétique.

2. Procédé de fabrication d'un réceptacle hermétiquement étanche selon la revendication 1, dans lequel le soudage de la feuille métallique (1) est exécuté en utilisant un laser à impulsions (5) pour former une pluralité de cordons de soudure connectés, formant ainsi le joint hermétique.

3. Procédé de fabrication d'un réceptacle hermétiquement étanche selon la revendication 1 ou la revendication 2, le procédé comprenant les étapes suivantes:
fournir un corps métallique (2) qui définit une ouverture (21) et qui comporte un rebord planaire (22);
refroidir et maintenir le corps métallique (2) à une température inférieure au point d'ébullition du gaz propulseur en aérosol ou de la formulation médicinale en aérosol contenant le gaz propulseur en aérosol à sceller dans le réceptacle;
introduire le gaz propulseur en aérosol liquéfié ou la formulation médicinale en aérosol contenant le gaz propulseur en aérosol liquéfié (10) dans le corps métallique (2);
positionner la feuille métallique (1) sur l'ouverture (21) et le rebord (22);
maintenir la feuille métallique (1) contre le rebord (22); et
souder la feuille métallique (1) au rebord (22) en utilisant un laser à impulsions (5) de manière à former une pluralité de cordons de soudure connectés autour du rebord (22), formant ainsi le joint hermétique, dans lequel l'étape d'introduction est exécutée au moins après l'étape de fourniture d'un corps métallique (2) et au moins avant l'étape de maintien, et dans lequel l'étape de refroidissement est exécutée au moins après l'étape de fourniture d'un corps métallique (2), et l'étape d'introduction est exécutée au moins après l'étape de refroidissement et au moins avant l'étape de maintien de la feuille métallique (1).

4. Procédé de fabrication d'un réceptacle hermétiquement étanche selon l'une quelconque des revendications précédentes, dans lequel le gaz propulseur en aérosol est sélectionné parmi le HFA 134a, le HFA 227 et des mélanges de ceux-ci.

5. Procédé de fabrication d'un réceptacle hermétiquement étanche selon l'une quelconque des revendications précédentes, dans lequel un gaz de protection est fourni autour de la soudure pendant le soudage.

6. Procédé de fabrication d'un réceptacle hermétiquement étanche selon la revendication 5 lorsqu'elle dépend directement ou indirectement de la revendication 3, dans lequel le gaz de protection est envoyé de manière à frapper la feuille (1) et est dirigé de manière à maintenir la feuille en contact intime avec le rebord (22).

7. Procédé de fabrication d'un réceptacle hermétiquement étanche selon l'une quelconque des revendications précédentes, dans lequel le volume interne du réceptacle étanche est de 2 ml ou moins.

8. Procédé de fabrication d'un réceptacle hermétiquement étanche selon l'une quelconque des revendications précédentes, dans lequel la feuille métallique (1) est constituée de métaux sélectionnés entre l'aluminium et l'acier inoxydable.

9. Procédé de fabrication d'un réceptacle hermétiquement étanche selon l'une quelconque des revendications 3 à 8, dans lequel le corps métallique (2) est constitué de métaux qui sont sélectionnés entre l'aluminium et l'acier inoxydable.

10. Procédé de fabrication d'un réceptacle hermétiquement étanche selon la revendication 9, dans lequel le corps métallique (2) et la feuille métallique (1) sont constitués d'acier inoxydable.
